# EUROPEAN PATENT SPECIFICATION

(11) **EP 1 844 661 B1**
(45) Date of publication and mention of the grant of the patent: **19.01.2011**
(21) Application number: 07105686.5
(22) Date of filing: 04.04.2007
(51) Int. Cl.: A23F 5/24

(54) **Stabilized enzyme compositions**
Stabilisierte Enzymzusammensetzungen
Composition d'enzymes stabilisée

(30) Priority: 10.04.2006 US 279157
(43) Date of publication of application: 17.10.2007
(73) Proprietor: Kraft Foods Global Brands LLC, Northfield, IL 60093 (US)
(72) Inventor: Silver, Richard Stuart, Wilmette, IL 60091 (US); Whalen-Pedersen, Erik, Spring Grove, IL 60081 (US)
(74) Representative: Smaggasgale, Gillian Helen

(56) References cited:
- EP-A- 1 745 702
- WO-A-2005/074705
- WO-A-2005/074707
- WO-A-2006/103515
- DE-A1- 1 692 266
- GB-A- 1 200 700
- US-A- 4 983 408
- US-A- 5 922 385
- US-A1- 2002 192 351
- US-B1- 6 664 070
- SACHSLEHNER A ET AL: "Hydrolysis of isolated coffee mannan and coffee extract by mannanases of Sclerotium rolfsii" JOURNAL OF BIOTECHNOLOGY, ELSEVIER SCIENCE PUBLISHERS, AMSTERDAM, NL, vol. 80, no. 2, 23 June 2000 (2000-06-23), pages 127-134, XP004213502 ISSN: 0168-1656
- FARDIAZ SRIKANDI: "Antimicrobial activity of coffee (Coffea robusta) extract" ASEAN FOOD JOURNAL, vol. 10, no. 3, 1995, pages 103-106, XP009088027 ISSN: 0127-7324
- VANITA MAHAJAN: "In vitro susceptibility of human pathogenic bacteria to coffee extracts." JOURNAL OF COFFEE RESEARCH 1993 DEP. OF BIOSCI., MAHRASHI DAYANAND UNIV., ROHTAK 124 001, HARYANA, INDIA, vol. 23, no. 2, 1993, page 103, XP009088061
- XU WEN ET AL: "Antioxidative activity of a zinc-chelating substance in coffee." BIOSCIENCE, BIOTECHNOLOGY, AND BIOCHEMISTRY 68 (11) 2313-2318 2004 CORRESPONDENCE (REPRINT) ADDRESS, S. HOMMA, DEP. OF NUTR. & FOOD SCI., OCHANOMIZU UNIV., BUNKYO-KU, TOKYO 112-8610, JAPAN. FAX +81-3-5978-5755. E-MAIL HOMMA(A)CC.OCHA.AC.JP, 2004, XP002446051

## Description

### FIELD OF THE INVENTION

This invention is directed to stabilized enzyme compositions. More specifically, this invention is directed to enzyme compositions stabilized with coffee-derived materials. The stabilized enzyme compositions are especially useful in the production of coffee products.

### BACKGROUND OF THE INVENTION

Food-grade enzymes are typically produced by microbial fermentation or by extraction from plant or animal tissues. The crude enzymes, which are typically in aqueous solution, may be purified and concentrated by techniques such as centrifugation, membrane ultrafiltration, microfiltration, salt precipitation, solvent precipitation, and the like. The refined enzymes are then standardized and formulated into a salable form, such as an aqueous liquid, a dried granulated powder, or an agglomerated granular form. Unformulated preparations - such as aqueous enzyme concentrates or salt precipitates - are often used for research purposes but are uncommon as commercial enzymes due to difficulty in handling, high probability of microbial growth, and/or decreased activity during storage. Stabilization of enzymes involves both inhibition of microbial growth and conservation of enzyme activity during processing and storage. Enzyme activity may decrease due to a number of natural processes, including oxidation, thermal hydrolysis, and proteolysis. Microbial growth is more common in aqueous systems and less of an issue in dry formulations. However, not all enzymes can easily be dried and in many cases aqueous formulations may be preferred due to the lack of airborne contaminants and the ease of use.

Certain food-approved materials or ingredients can be added to liquid or dried enzyme formulations in order to (1) stabilize enzyme activity during purification, processing, storage, and/or abuse, (2) inhibit the growth of microorganisms, (3) permit standardization of the active ingredient, and/or (4) facilitate more convenient dispensing of the enzyme. Such materials or ingredients include antimicrobials which inhibit microbial growth in the product. stabilizers which preserve enzyme activity during drying, storage, or abuse, dispersants which help wet or disperse the enzyme, buffering agents which control the pH of the formulation and during use and bulking/standardization agents which permit adjustment of activity to a standardized level and convenient dispensing of the enzyme. Examples of food-grade antimicrobials include sorbic acid, propionic acid, potassium sorbate, sodium erythorbate. Other stabilizers include sugars such as sucrose and lactose, polyols such as glycerol, sorbitol, mannitol, salts such as sodium chloride, potassium chloride, ammonium chloride, organic acids such as citric acid, proteinaceous materials such as non-fat milk, and whey protein concentrate, and the like, oligomers such as polyvinylpyrrolidone, polyethylene glycol, and amphoteric materials such as betaine and gamma-aminodibutyrate. The mechanism (mode of action) of the stabilization is not always well known; but appears to involve protection of the enzyme protein (or in some cases, non-protein entity required for activity) from the denaturing or deactivating of thermal extremes, desiccation, shear, osmotic stress, pH stress, ionic gradients, proteolysis and other adverse forces. Buffering agents include acetic acid, sodium acetate, sodium phosphate, sulfuric acid, calcium carbonate and the like. Dispersants include lecithin, saponins, and the like. If the enzyme preparation is to be dried, it may be preferred to add the formulation or stabilizing ingredients to the aqueous liquid prior to drying since they are better mixed with the enzymes and the stabilizers can protect the enzymes during drying.

Soluble coffee, also referred to as instant coffee, is a convenient alternative to the more traditional roast and ground coffee (R&G). Instant coffees, however, often trade the robust flavor of the R&G bean for the convenience of quick preparation. Soluble coffee is typically made by extraction and thermal hydrolysis of roast and ground (R&G) coffee, followed by separation and drying of the extract. Often soluble coffees may have an imbalanced flavor and aroma due to the high degree of processing and associated losses. The high temperatures and pressures used in such processes often produce undesirable off-flavors, thereby generating a lesser quality product as compared to traditional R&G coffee.

The inferior quality of the soluble coffees has been a longstanding problem. Various attempts have been made to design a high-yield, soluble-coffee process that eliminates undesirable off-flavors and retains as much of the coffee flavor and aroma as possible. The use of enzymes in the production of soluble coffee has been explored in an attempt to solve this longstanding problem.

For example, U.S. Patent No. 2,282,138 to Kellogg describes the production of soluble coffee using a converting enzyme, such as taka diastase, to treat the coffee material. This process uses high pressure steam (15 psi) to soften and loosen the coffee bean matrix followed by cooling and subsequent treatment with the enzyme at temperatures not substantially above 65°C and typically between 48 and 54°C.

U.S. Patent 4,983,408 to Colton describes a process that uses enzymes to solubilize coffee components after subjecting ground coffee to a "steam explosion" pretreatment followed by a rapid decompression to atmospheric pressures. Enzymes used include amylases, hemicellulase, cellulase, protease, cellobiase, pectinase, and lipases.

U.S. Patent No. 4,904,484 to Small et al. describes a method to produce R&G coffee by treating green or partially roasted coffee beans with enzymes prior to a final roasting. The enzymes used include cell-wall-digesting, cell-storage-component-digesting, or phenol oxidase enzymes. Cell-wall digesting enzymes include cellulases, hemicellulases, pectinases, glucanases, mannases, and ligninases. Cell-storage-component-digesting enzymes include amylases, glucosidases, mannosidases, dextranases, and proteases. Phenol oxidase enzymes include tyrosinase, phenolase, and other extracts such as teas, apple juice, pear juice, and grape juice.

The use of coffee materials such as spent coffee grounds as a fermentation nutrient for the microbial production of enzymes has been reported. See, e.g., Regalado et al., J. Sci. Food Agr., 80:9, pp. 1343-1350 (2000).

More recently, soluble coffee extracts prepared using enzymes in a hydrolysis process coupled with membrane permeation technologies have been prepared (see Rep 1745702).
A roasted coffee solid is finely wet milled to form a coffee slurry. The coffee slurry is treated with an effective amount of a suitable enzyme to form a reaction mixture and then treated at about 20 to about 90°C to hydrolyze coffee components of the roasted coffee solids to form the soluble coffee material. The soluble coffee material is extracted with water and then separated into a retentate, which comprises the enzyme and other insoluble particulates, and a permeate, which comprises the purified soluble coffee extract, using a semi-permeable membrane. Enzymes especially useful in this process include hydrolases such as mannanase, cellulase, hemicellulase, protease, pectinase, nuclease, and lipase. This process provides a soluble coffee having a more balanced, superior flavor without undesired thermal degradation compounds as compared to the thermal process while maintaining comparable solubilization yield.

While enzymes make it possible to overcome many of the limitations of the soluble coffee manufacturing art by significantly reducing the heat input to which the products are exposed, their incorporation in the coffee processes may carry a degree of complexity. The enzyme proteins themselves are high molecular weight and will be removed by the soluble coffee process, but many of the typical formulation or stabilization ingredients are low molecular weight and will persist in the final product. Specifically, ingredients used to stabilize the enzymes activity during shelf life storage may be carried over into the finished products and may cause undesirable quality side effects. For example, although the process described in EP1745702 uses membrane separation, such formulation ingredients may pass through the membrane and be concentrated in the soluble coffee product. Even such initially insoluble formulation agents such as maltodextrins may be partially solubilized by the enzyme process and appear in the coffee product. Removal of such formulation agents will also increase the complexity and cost of the process.

Consequently, there remains a need to provide an efficient method of producing soluble coffee that retains the robust coffee flavor and aroma similar to R&G coffee using enzymes but without conventional formulation ingredients normally used to stabilize such enzyme formations. Moreover, there remains a need to provide a simplified method of soluble coffee production that reduces costs and unit operations.

### SUMMARY OF THE INVENTION

This invention is directed to stabilized enzyme compositions. More specifically, this invention is directed to enzyme compositions stabilized with coffee-derived materials. The stabilized enzyme compositions of this invention have comparable stability of the active components as conventionally stabilized formulations but only contain coffee-derived formulation ingredients. The stabilized enzyme compositions of this invention can be used in any application where it is desired to avoid inclusion of conventional stabilizers and other stability-enhancing additives. The stabilized enzyme compositions are especially useful in the enzymatic production of soluble coffee and other coffee products.

The present invention provides in a first aspect a stabilized enzyme composition comprising an enzyme and an effective amount of a coffee-derived material to stabilize the enzyme, wherein the composition is in a dried form and wherein the composition contains about 1 to about 25 percent enzyme and about 75 to about 99 percent coffee-derived material.

The present invention also provides a method to produce a soluble coffee extract, said method comprising:
(1) treating coffee solids to form a coffee slurry containing coffee solids;
(2) treating the coffee slurry with an effective amount of an enzyme in the form of a stabilized enzyme composition at a temperature and for a time sufficient to hydrolyze the coffee solids to form a soluble coffee extract material, wherein the stabilzed enzyme composition is the composition of the above first aspect; and
(3) treating the soluble coffee extract material to obtain the soluble coffee extract.

This invention also provides a semi-continuous or continuous method to produce a soluble coffee extract, said method comprising:
(1) treating finely-ground roasted coffee solids with an effective amount of an enzyme in the form of a stabilized enzyme composition in a vessel to hydrolyze the coffee solids to form a soluble coffee extract material containing soluble coffee extract, wherein the stabilized enzyme composition is the composition of the above first aspect;
(2) circulating the soluble coffee extract material through a semi-permeable membrane separation device to continuously form a retentate and a permeate, wherein the permeate contains the soluble coffee extract and is continuously collected and wherein at least a portion of the retentate is recycled to the vessel.

### DETAILED DESCRIPTION

As noted, this invention is directed to enzyme compositions stabilized with coffee-derived materials. The stabilized enzyme compositions of this invention can be used in any application where it is desired to avoid inclusion or use of conventional stabilizers and other stability-enhancing additives. Thus, the present stabilized enzyme compositions can be used in the production of various food products, pharmaceutical products, cosmetic products, and the like so long as the coffee-derived materials are acceptable during processing and in the final product. These stabilized enzyme compositions avoid conventional stabilizers while still maintaining the desired enzyme stability and activity during storage and/or abuse using only coffee-derived materials.

The stabilized enzyme compositions are especially useful in the production of soluble coffee and other coffee products using enzymes which do not contain conventional stabilizers normally associated with enzyme formulations. Thus, the stabilized enzyme compositions of this invention are especially useful in an enzyme treatment of the coffee solids to hydrolyze the coffee materials therein.

The present invention can also be used to stabilize enzyme preparation useful in food, pharmaceutical, cosmetic, and other industries where conventional stabilizers are undesirable. Enzymes especially useful in food preparation which can be stabilized using this method include mannanase, cellulase, glucanase, hemicellulase, lipase, esterase, protease, carbohydrase (e.g., arabinase, galactanase, arabino-galactanase), nuclease, pectinase, isomerase, amylase, and ligninase as well as mixtures thereof. Using the present coffee-derived material stabilized enzyme compositions, soluble coffee products can easily be prepared without the use of conventional enzyme stabilizers.

The coffee-derived materials useful for stabilizing enzyme compositions include, for example, soluble coffee, roast and ground coffee, coffee oils, spent (i.e., partially extracted) coffee grounds, ground green coffee beans, aqueous coffee extract, green coffee bean extracts, and the like as well as mixtures thereof; the extracts or other coffee materials may be concentrated if desired. Of course, such coffee-derived materials should not include, or be prepared with enzyme compositions containing, the conventional stabilizer which the present invention seeks to eliminate from the final product. Thus, for example, soluble coffee materials used to stabilize the present enzyme compositions should not be derived from soluble coffee prepared using conventionally stabilized enzyme compositions.

The stabilized enzyme compositions of the present invention is dried to form solid compositions (e.g., powder or agglomerated particles).
For dry formulations, the active enzyme protein comprises about 1 to about 25 percent of the total formulation and the coffee-derived materials comprise about 75 to about 99 percent of the total formulation. For dried forms, the coffee-derived materials are preferably present during the formation of the dried enzyme composition (for example, by spray drying, freeze drying, and the like).
Dried compositions generally may be stored under ambient, refrigerated, or frozen conditions.

For purposes of this invention, "conventional stabilizers" include conventional antimicrobials which inhibit microbial growth in the product and conventional stabilizers which preserve enzyme activity during drying, storage, or abuse. Although not wishing to be limited by theory, it appears that the coffee-derived materials used in the present invention perform both of these functions (i.e., inhibiting microbial growth and preserving enzyme activity during storage and/or abuse). Again not wishing to be limited by theory, it also appears that the coffee-derived materials also have other functions such as, for example, buffering agents and dispersants in the enzyme formulation. Thus, it appears that the coffee-derived stabilizers of this invention provides protection of the enzyme protein (or in some cases, non-protein entity required for activity) from the denaturing or deactivating of thermal extremes, desiccation, shear, osmotic stress, pH stress, ionic gradients, proteolysis and other adverse forces.

When the stabilized enzymes of this invention are used to prepare soluble coffee extract, the unpurified extract material comprises soluble coffee extract, enzyme, and other insoluble particulates. Of course, since the enzyme composition was stabilized with coffee-derived materials, no conventional stabilizers are present and the enzyme and other insoluble particulate can be separated by conventional means to obtain soluble coffee extract essentially free of non-coffee materials. The desired coffee extract can preferably be separated from the other components by passing the reaction mixture over a semi-permeable membrane as described, for example, in EP1745702.
The membrane may be any type of material with a pore size that is capable of retaining the enzyme and other insolubles while permitting the permeation of water soluble saccharides, coffee extract, and other materials. Generally, membranes having a nominal pore size of less than about 0.8 microns would be suitable. For example, a membrane made out of polyethersulfone with a molecular weight cut-off of about 20,000 to about 50,000 Daltons is suitable to separate the soluble coffee extract. A preferred polyethersulfone membrane suitable for use in the method of this invention is available from Sepro, Inc. (Oceanside, CA). Of course, other material-of-construction membranes may be used. The membrane may be contained inside a cross-flow-membrane separation cell or other similar device that may be used for membrane separation, such as spiral wound modules, hollow-fiber systems, tubular bundles, or plate and frame modules.

Advantages and embodiments of this invention are further illustrated by the following examples but the particular materials and amounts thereof recited therein, as well as other conditions and details, should not be construed to unduly limit the invention. All parts, ratios, and percentages are by weight unless otherwise directed.

**Example 1.** A frozen, unformulated mannanase enzyme (FUM: ChemGen CL160 from ChemGen Corp., Gaithersburg, MD) was used. The FUM enzyme was from a *Bacillus lentus* fermentation; cells and debris were removed by centrifugation followed by enzyme concentration and purification using membrane ultrafiltration; no other materials were added. Thawed enzyme was diluted 1:20 in (sample 1) deionized water and (sample 2) deionized water containing 10% Kenco^{®} Really Rich retail soluble coffee. Samples of both solutions were immersed in a boiling water bath for 30 seconds and then immediately chilled in an ice bath. The treated solutions, as well as an unheated solution of sample 1 (i.e., control), were assayed for mannanase activity using a viscometric assay with carob gum. As compared to the control, treated sample 1 lost about 33% of the initial enzyme activity whereas sample 2, stabilized with coffee-derived materials, lost only about 2% of the initial enzyme activity (i.e., essentially unchanged activity within experimental limits).

**Example 2.** This example illustrates the preparation of dry enzyme formulations stabilized by coffee-derived materials. Frozen unformulated mannanase enzyme ("FUM") enzyme from Example 1 was used. A portion of the frozen FUM was thawed and five dry (solid) formulations were prepared by adding the following individual ingredients (at 20 percent) to the thawed FUM samples:
(1) Soluble coffee (Maxwell House^{®});
(2) Sorbitol and sodium chloride (10% each);
(3) Silica (40-100 mesh; Fisher Scientific);
(4) Cellulose (Neocel™ powder; Mingtai Chemical); and
(5) Roast and Ground (R&G) coffee (Maxwell House^{®}, dry milled to -30 mesh).
Samples 2-4 were formulated with typical stabilizers used in enzyme formulations. Each preparation was well mixed and then immediately freeze dried in a VirTis Model 25ES freeze drier. Portions of the freeze dried formulations were stored at 20°C and 50°C; samples were withdrawn at different time intervals for enzyme activity and microbiological assays.

Enzyme activity was measured using a viscometric assay using a freshly prepared aqueous solution of locust bean gum (1%: initial viscosity of 2800-3500 cP at 20°C). The enzyme formulation was diluted 1:10 in water, well-mixed, and then further diluted as required. Diluted samples were assayed promptly after preparation. To a tube containing 30 ml of gum solution at 20°C, 25 microliters of a suitable dilution of a given enzyme solution was added at "time zero." A suitable dilution (generally about 1:100) of the enzyme formulation was effective to reduced the gum viscosity to <500 cP in about 5-15 minutes.

Each gum-enzyme solution was mixed for about 30 seconds and then analyzed on a Brookfield DVII+ viscometer (Spindle 6 at 20 RPM). The viscosity versus time was recorded using Brookfield Wingather software. The relative enzyme activity was determined as the linear-fit slope of the viscosity versus time curve to 500 cP and/or the time required to reach 500 cP. Since slope response was not necessarily linear with enzyme dilution, comparative samples and reference controls were preferably measured at the same dilution (generally about 1:100). Due to the concentration factor involved in freeze drying, the dry samples, assuming no loss of activity, should have about 2.5-fold the activity per unit weight (i.e., 250) of the initial FUM sample (i.e., 100). The results are shown in Table 1 below.

**Table 1.**

| Formulation Additive | Relative Activity* | |
|---|---|---|
| | Storage at 20°C for 87 days | Storage at 50°C for 56 days |
| Soluble coffee | 271 | 167 |
| Sorbitol & sodium chloride^{†} | 198 | 118 |
| Silica^{†} | 235 | 49 |
| Cellulose^{†} | 159 | 35 |
| R8G coffee | 272 | 163 |

| | | |
|---|---|---|
| * Relative activity of FUM is 100. Formulations may have an activity greater than 100 due to concentration effects. ^{†} Conventional stabilizers used In enzyme formulations. | | |

Coffee-material stabilized samples stored at 20°C surprisingly have somewhat higher activity than the other formulations even after almost 3 months storage.

Formulations stored at the abusive temperature of 50°C were significantly less active than those stored at 20°C. However, surprisingly the coffee-based formulations retained a higher percentage of activity (i.e., over about 60% of the activity remaining at storage of 20°C) as compared to the non-coffee material formulations. Therefore, coffee-derived materials in dried formulations served as a stabilizer at storage temperatures at 20° and 50°C.

Microbiological stability was evaluated by determining the microbial total plate counts (TPC) using AOAC method 966.23. The initial frozen FUM had TPC of 9800 counts/gm. The freeze drying process increased the dry matter concentration by 2.5 fold. Table 2 shows microbial TPC in samples stored for 3 and 9 weeks at 20°C and for 3 weeks at 50°C.

**Table 2**

| Formulation Additive | Total Plate Counts/g* | | |
|---|---|---|---|
| | Storage at 20°C | | Storage at 50°C for |
| | 3 weeks | 9 weeks | 56 days |
| Soluble coffee | 6300 | <300 | 240 |
| Sorbitol & sodium chloride^{†} | 10,000 | <300 | 750 |
| Silica^{†} | 5500 | <300 | 500 |
| Cellulose^{†} | 500 | <300 | 100 |
| R&G coffee | 6500 | <300 | 380 |

| | | | |
|---|---|---|---|
| *Reference FUM liquid had a total plate counts/g of 9800 ^{†}Conventional stabilizers used in enzyme formulations. | | | |

All dried samples showed a significant reduction in TPC (as opposed to an increase due to the concentration factor only). The samples formulated with coffee materials were comparable to other formulation materials. Samples stored at 50°C had much lower counts than those stored at 20°C. The dry samples stored at 20°C for 9 weeks all assay at less than 300 TPC/gm. These dry samples have very low water activity so microbial growth is inhibited. All dried samples have acceptably low microbial TPC.

**Example 3.** This example illustrates the preparation of liquid enzyme formulations stabilized by coffee-derived materials. The frozen unformulated mannanase enzyme of Example 1 was thawed and liquid formulations were prepared by adding the following individual ingredients (at 20 percent):
(1) Soluble coffee (Maxwell House^{®});
(2) Sorbitol and sodium chloride (20% each); and
(3) Control FUM (no additives).
Portions of the aqueous liquid formulations were stored at 4, 20, and 50°C and samples withdrawn at different time intervals for enzyme activity and microbiological assay using the same analytical techniques as in Example 1. The enzyme activity and microboilogical results are shown in Table 3 and 4, respectively.

**Table 3.**

| Formulation Additive | Relative Activity | | |
|---|---|---|---|
| | 4°C for 49 days | 20°C for 60 days | 50°C for 56 days |
| Control | 97.5 | 31.9 | 11.1 |
| Sorbitol & sodium chloride^{†} | 47.6 | 32.3 | 19.0 |
| Soluble Coffee | 73.4 | 10.0 | 0 |

| | | | |
|---|---|---|---|
| ^{†} Conventional stabilizers used in enzyme formulations. | | | |

**Table 4**

| Formulation Additive | Total Plate Counts/g | | |
|---|---|---|---|
| | 4°C for 3 weeks | 20°C for 3 weeks | 50°C for 3 weeks |
| Control | 3700 | 9.5 x 10⁷ | 80 |
| Sorbitol & sodium chloride^{†} | 3600 | 530 | 270 |
| Soluble coffee | 100 | 200 | 20 |

| | | | |
|---|---|---|---|
| ^{†} Conventional stabilizers used in enzyme formulations. | | | |

The enzyme activities for the liquid samples stored at different times and temperatures are shown in Table 3. Due to the dilution effect of the formulation ingredients, the formulated liquids would be predicted to have about 60% of the activity of control (i.e., unformulated FUM) assuming no effect for the additives.

For samples stored at 4°C, unformulated FUM retained a high percentage of control activity (i.e., over 97%) even after over 7 weeks storage. Surprisingly, the soluble coffee formulation stored at 4°C for over 7 weeks had over 73% of control activity; this is higher than the predicted 60% if the ingredients had no effect. The salt/sorbitol formulation stored at 4°C had 47.6% of control activity.

For storage under abusive conditions of 50°C for 8 weeks, the unformulated FUM retained only 11% of control activity. The soluble coffee formulation formed a virtually insoluble gel at 50°C and lost essentially all activity. For 20°C storage, although there was substantial microbial growth in the unformulated FUM (see below), about 32% of control enzyme activity was retained; this material would be unacceptable due to the microbial growth. The soluble coffee formulation retained only 10% of control activity and a partially insoluble gel was formed.

Based on this data, soluble coffee appears to be an effective enzyme activity stabilizer in aqueous liquid formulations stored at refrigerated conditions (4°C). It is not, however, effective for room temperature (20°C) storage or for more abusive conditions (50°C).

As shown in Table 4, the unformulated control FUM was microbially unstable when stored at 20°C; in 3 weeks time the total plate count had increased dramatically. However, the unformulated control stored at 4°C showed over 60% reduction in TPC and 99% when stored at 50°C. This is presumably due to death during storage of a portion of the microbes initially present. Conversely, both formulated samples showed good microbial stability when stored at 20°C for 3 weeks, with the soluble coffee formulation giving somewhat lower TPC. Therefore, 40 wt.% soluble coffee inhibits microbial growth during 20°C storage. At 6 weeks storage at 20 °C, the formulated samples showed little change in TPC.

## Claims

1. A stabilized enzyme composition comprising an enzyme and an effective amount of a coffee-derived material to stabilize the enzyme, wherein the composition is in a dried form and wherein the composition contains about 1 to about 25 percent enzyme and about 75 to about 99 percent coffee-derived material.

2. The composition of Claim 1, wherein the enzyme is a hydrolase enzyme.

3. The composition of Claim 1 or 2, wherein the hydrolase enzyme is selected from the group consisting of mannanase, cellulase, glucanase, hemicellulase, lipase, esterase, protease, arabinase, galactanase, arabino-galactanase, nuclease, pectinase, isomerase, amylase, ligninase, and mixtures thereof.

4. The composition of any one of Claims 1 to 3, wherein the coffee-derived material is .. selected from the group consisting of soluble coffee, roast and ground coffee, coffee oil, spent coffee grounds, ground green coffee beans, aqueous coffee extract, green coffee bean extract, and mixtures thereof.

5. A method to produce a soluble coffee extract, said method comprising:
(1) treating roasted coffee solids to form a coffee slurry containing coffee solids;
(2) treating the coffee slurry with an effective amount of an enzyme in the form of a stabilized enzyme composition at a temperature and for a time sufficient to hydrolyze the coffee solids to form a soluble coffee extract material, wherein the stabilized enzyme composition is the composition of any one of Claims 1 to 4; and
(3) treating the soluble coffee extract material to obtain the soluble coffee extract.

6. The method of Claim 5, wherein the coffee slurry in step (1) is formed by wet milling the roasted coffee solids and wherein the soluble coffee extract in step (3) is obtained by separating the soluble coffee extract material into a retentate and a permeate, wherein the permeate comprises the soluble coffee extract.

7. The method of Claim 6, wherein the separation of the soluble coffee extract material into a retentate and a permeate is carried out using a semi-permeable membrane having a molecular weight cut-off from about 20,000 to about 50,000 Daltons.

8. A semi-continuous or continuous method to produce a soluble coffee extract, said method comprising:
(1) treating finely-ground coffee solids with an effective amount of an enzyme in the form of a stabilized enzyme composition to hydrolyze the coffee solids to form a soluble coffee extract material containing soluble coffee extract, wherein the stabilized enzyme composition is the composition of any one of Claims 1 to 4;
(2) circulating the soluble coffee extract material through a semi-permeable membrane separation device to continuously form a retentate and a permeate, wherein the permeate contains the soluble coffee extract and is continuously collected and wherein at least a portion of the retentate is recycled to the vessel.

9. The method of Claim 8, wherein the semi-permeable membrane has a molecular weight cut-off from about 20,000 to about 50,000 Daltons.

## Patentansprüche

1. Stabilisierte Enzymzusammensetzung mit einem Enzym und einer wirksamen Menge eines von Kaffee abstammenden Materials zur Stabilisierung des Enzyms, wobei die Zusammensetzung in einer getrockneten Form ist und etwa 1 bis etwa 25 Prozent Enzym und etwa 75 bis etwa 99 Prozent von Kaffee abstammendes Material enthält.

2. Zusammensetzung des Anspruchs 1, bei der das Enzym ein Hydrolaseenzym ist.

3. Zusammensetzung des Anspruchs 1 oder 2, bei der das Hydrolaseenzym aus der Gruppe ausgewählt ist, die aus Mannanase, Cellulase, Glucanase, Hemicellulase, Lipase, Esterase, Protease, Arabinase, Galactanase, Arabino-Galactanase, Nuclease, Pectinase, Isomerase, Amylase, Ligninase und deren Gemischen besteht.

4. Zusammensetzung eines der Ansprüche 1 bis 3, bei der das von Kaffee abstammende Material aus der Gruppe ausgewählt ist, die aus löslichem Kaffee, gemahlenem Röstkaffee, Kaffeeöl, verbrauchten Kaffeemehlen, gemahlenen grünen Kaffeebohnen, wässrigem Kaffeeextrakt, Extrakt grüner Kaffeebohnen und deren Gemischen besteht.

5. Verfahren zur Herstellung eines löslichen Kaffeeextrakts, bei dem man
(1) geröstete Kaffeefeststoffe behandelt, um eine Kaffeefeststoffe enthaltende Kaffeeaufschlämmung zu bilden,
(2) die Kaffeeaufschlämmung mit einer wirksamen Menge eines Enzyms in der Form einer stabilisierten Enzymzusammensetzung bei einer ausreichenden Temperatur und einer genügende Zeit behandelt, um die Kaffeefeststoffe unter Bildung eines löslichen Kaffeeextraktmaterials zu hydrolysieren, wobei die stabilisierte Enzymzusammensetzung die Zusammensetzung eines der Ansprüche 1 bis 4 ist, und
(3) das lösliche Kaffeeextraktmaterial behandelt, um den löslichen Kaffeeextrakt zur erhalten.

6. Verfahren des Anspruchs 5, bei dem die Kaffeeaufschlämmung in Stufe (1) durch Naßmahlung der gerösteten Kaffeefeststoffe gebildet wird und der lösliche Kaffeeextrakt in Stufe (3) durch Trennung des löslichen Kaffeeextraktmaterials in ein Retentat und ein Permeat erhalten wird, wobei das Permeat den löslichen Kaffeeextrakt enthält.

7. Verfahren des Anspruchs 6, bei dem die Trennung des löslichen Kaffeeextraktmaterials in ein Retentat und ein Permeat unter Benutzung einer semipermeablen Membran mit einer Molekulargewichtsgrenze von etwa 20.000 bis etwa 50.000 Dalton durchgeführt wird.

8. Halbkontinuierliches oder kontinuierliches Verfahren zur Herstellung eines löslichen Kaffeeextrakts, bei dem man
(1) feingemahlene Kaffeefeststoffe mit einer wirksamen Menge eines Enzyms in der Form einer stabilisierten Enzymzusammensetzung behandelt, um die Kaffeefeststoffe unter Bildung eines löslichen Kaffeeextrakt enthaltenden löslichen Kaffeeextraktmaterials zu hydrolysieren, wobei die stabilisierte Enzymzusammensetzung die Zusammensetzung eines der Ansprüche 1 bis 4 ist, und
(2) das lösliche Kaffeeextraktmaterial durch eine Trenneinrichtung mit semipermeabler Membran zirkuliert, um kontinuierlich ein Retentat und ein Permeat zu bilden, wobei das Permeat den löslichen Kaffeeextrakt enthält und kontinuierlich gesammelt wird und wenigstens ein Teil des Retentat zu dem Behälter zurückgeführt wird.

9. Verfahren des Anspruchs 8, bei dem die semipermeable Membran eine Molekulargewichtsgrenze von etwa 20.000 bis etwa 50.000 Dalton hat.

## Revendications

1. Composition enzymatique stabilisée comprenant une enzyme et une quantité efficace d'une matière dérivée du café pour stabiliser l'enzyme, dans laquelle la composition est sous une forme séchée et dans laquelle la composition contient environ 1 à environ 25 % d'enzyme et environ 75 à environ 99 % de matière dérivée du café.

2. Composition selon la revendication 1, dans laquelle l'enzyme est une enzyme d'hydrolase.

3. Composition selon la revendication 1 ou 2, dans laquelle l'enzyme d'hydrolase est choisie dans le groupe constitué par mannanase, cellulase, glucanase, hémicellulase, lipase, estérase, protéase, arabinase, galactanase, arabino-galactanase, nucléase, pectinase, isomérase, amylase, ligninase et mélanges de celles-ci.

4. Composition selon l'une quelconque des revendications 1 à 3, dans laquelle la matière dérivée du café est choisie dans le groupe constitué par le café soluble, le café torréfié et moulu, l'huile de café, le marc de café, les grains de café vert moulus, l'extrait aqueux de café, l'extrait de grains de café vert et des mélanges de ceux-ci.

5. Procédé pour produire un extrait de café soluble, ledit procédé comprenant :
(1) le traitement des matières sèches de café torréfié pour former une bouillie de café contenant des matières sèches de café ;
(2) le traitement de la bouillie de café avec une quantité efficace d'une enzyme sous la forme d'une composition enzymatique stabilisée à une température et pendant un temps suffisants pour hydrolyser les matières sèches de café pour former une matière d'extrait de café soluble, dans lequel la composition enzymatique stabilisée est la composition selon l'une quelconque des revendications 1 à 4 ; et
(3) le traitement de la matière d'extrait de café soluble pour obtenir l'extrait de café soluble.

6. Procédé selon la revendication 5, dans lequel la bouillie de café dans l'étape (1) est formée par mouture humide des matières sèches de café torréfié et dans lequel l'extrait de café soluble de l'étape (3) est obtenu en séparant la matière d'extrait de café soluble en un rétentat et un perméat, le perméat comprenant l'extrait de café soluble.

7. Procédé selon la revendication 6, dans lequel la séparation de la matière d'extrait de café soluble en un rétentat et un perméat est opérée en utilisant une membrane semi-perméable ayant un seuil de coupure en masse moléculaire d'environ 20 000 à environ 50 000 Daltons.

8. Procédé semi-continu ou continu pour produire un extrait de café soluble, ledit procédé comprenant :
(1) le traitement des matières sèches de café finement moulues avec une quantité efficace d'une enzyme sous la forme d'une composition enzymatique stabilisée pour hydrolyser les matières sèches de café pour former une matière d'extrait de café soluble contenant un extrait de café soluble,
dans lequel la composition enzymatique stabilisée est la composition selon l'une quelconque des revendications 1 à 4 ;
(2) la mise en circulation de la matière d'extrait de café soluble à travers un dispositif de séparation à membrane semi-perméable pour former en continu un rétentat et un perméat, le perméat contenant l'extrait de café soluble et étant collecté en continu, et au moins une partie du rétentat étant recyclée vers le récipient.

9. Procédé selon la revendication 8, dans lequel la membrane semi-perméable a un seuil de coupure en masse moléculaire d'environ 20 000 à environ 50 000 Daltons.
